# EUROPEAN PATENT APPLICATION

(11) **EP 3 838 398 A1**
(43) Date of publication of application: **23.06.2021**
(21) Application number: 19218435.6
(22) Date of filing: 20.12.2019
(51) Int. Cl.: B01J 19/24, B01J 19/00, B01J 3/04, B01J 4/02, B01J 10/00, C07C 273/12, C07D 251/62

(54) **COMBINED REACTOR FOR HIGH-PRESSURE SYNTHESIS OF MELAMINE**

(71) Applicant: CASALE SA, 6900 Lugano (CH)
(72) Inventor: GAMBA, Simone, 24040 Pagazzano (BG) (IT); DI CARLO, Gabriele, 6900 Lugano (CH); RIZZI, Enrico, 22070 Casnate con Bernate (CO) (IT)
(74) Representative: M. Zardi & Co S.A.

(57) **Abstract**

Reactor for the high-pressure non-catalytic synthesis of melamine from urea, comprising coaxial inner reaction zone (6) and outer reaction zone (7) wherein a crude melamine is formed in the inner reaction zone and contacted with gaseous ammonia for stripping in the outer reaction zone, wherein a gaseous phase liberated in the outer zone is collected in a gas collection chamber (12) above the reaction zones, wherein the crude melamine melt is transferred from the inner zone into the outer zone via a submerged liquid passage below the liquid level to provide a liquid seal between the chambers.

## Description

### Field of application

The invention relates to plants for the synthesis of melamine from urea. The invention relates in particular to reactors for the high-pressure synthesis of melamine.

### Prior art

The processes for the synthesis of melamine from urea are commonly classified as low-pressure catalytic processes and high-pressure non-catalytic processes. A low-pressure process is typically conducted at a pressure below 1 MPa; a high pressure process is generally conducted at a pressure of at least 7 MPa and preferably in the range 7 to 25 MPa. The low-pressure process and the high-pressure process are described in the literature, example Ullmann's Encyclopedia of Industrial Chemistry, 6th ed., vol. 21, p. 205.

The production of melamine can be integrated with the production of urea, because urea is the source material for the synthesis of melamine and the synthesis of melamine produces offgas containing ammonia and carbon dioxide, which are the starting materials for the production of urea. Therefore melamine can be produced in a so-called integrated urea-melamine facility including a melamine plant and a tied-in urea plant.

A known high-pressure melamine synthesis process comprises essentially three steps: a bulk conversion of urea into a crude melamine melt; a second step of removal of the carbon dioxide (CO₂) contained in the melamine melt by introducing gaseous ammonia, and reduction of the content of by-products which are converted into melamine; a third step during which the gases formed in the first two steps are washed or scrubbed with urea for subsequent recycle to the urea synthesis section. The above three steps are conventionally performed in separate vessels called respectively primary reactor, secondary reactor or stripping reactor, and scrubber.

A setup with three separate pressure vessels however has disadvantages in terms of cost and complication.

WO 2015/124409 discloses a combined apparatus for the synthesis of melamine with an inner reaction space and an outer reaction space coaxially arranged, wherein the inner reaction space performs the function of a primary reactor and the outer reaction space performs the function of a secondary reactor. The inner reaction space is delimited by an internal shell of the reactor. The reactor may include an upper dome acting as scrubber.

The above described coaxial reactor operates as follows. The bulk conversion of urea to melamine and formation of the crude melamine melt takes place in the inner reaction space; the crude melamine melt formed in the inner space flows into the outer reaction space by overflowing from the upper edge of the internal shell; in the outer reaction space, gaseous ammonia is added as a stripping medium; the by-products formed during the bulk conversion are converted into melamine and CO₂ is removed together with gaseous ammonia.

### Summary of the invention

The invention aims to improve the above mentioned prior art of a combined reactor including an inner reaction space and an outer reaction space coaxially arranged. More specifically, the invention aims to reduce the byproducts contained in the melamine withdrawn from the reactor and therefore to increase its purity.

The present invention is based on the finding that in a reactor with the above described arrangement of inner and outer coaxial reaction spaces, the gas mixture collected in the upper part of the reactor and above the coaxial reaction spaces contains a significant amount of CO₂ generated during the bulk conversion of urea to melamine which occurs in the inner reaction space. The applicant has found that this CO₂ collected above the coaxial reaction spaces can diffuse into the outer reaction space and affect its performance of secondary conversion, namely the conversion of byproducts into melamine and removal of CO₂. If the conversion of byproducts and/or the removal of CO₂ are affected, the purity of the final melamine-containing product is also affected.

Based on this finding, the above aim is reached with a reactor and a process for the synthesis of melamine according to the claims.

In the inventive reactor and process, the primary bulk conversion and the secondary conversion are performed, respectively, in an inner reaction space and an outer reaction space coaxially arranged. The liquid crude melamine melt formed in the inner reaction space is transferred to the outer reaction space via a passage which, during operation, is located below the liquid level of said melamine melt.

Due to the above described immersed location of the melamine melt passage, a hydraulic seal is created between the inner reaction space and the outer reaction space. A differential pressure is established between the outer reaction space and the inner reaction space. Particularly, the outer reaction space operates at a higher pressure than the inner reaction space. The melamine melt from the inner reaction space can flow into the outer reaction space by creating a static head; this liquid head provides a hydraulic seal between the two reaction spaces.

In addition, and according to preferred embodiments, the outlet area for the extraction of gas from the outer reaction space can be reduced in order to increase the gas outlet velocity. The faster gas output increases the convective flow of the outlet gas; consequently, the diffusive transfer of gaseous carbon dioxide into the outer reaction zone is reduced.

The invention has therefore the following advantages: the gaseous CO₂ collected at the top of the reactor, above the coaxial reaction spaces, is substantially prevented from entering the outer reaction space where the secondary conversion is performed; the available reaction volume is exploited at its best; the purity of the obtained melamine is increased because the conversion of byproducts into melamine is no longer affected by the undesired diffusion of CO₂.

### Description of preferred embodiments

A reactor according to the invention includes generally an outer pressure shell; a urea input arranged to direct a urea feed into the first reaction zone; separation means arranged to separate the first reaction zone from the second reaction zone.

The reactor includes at least one communication port between the first reaction zone and the second reaction zone, said port being arranged to transfer liquid crude melamine melt formed in the first reaction zone to the second reaction zone, where the melamine melt is further processed.

The reactor further includes means for withdrawing a melamine-containing product from bottom of the second reaction zone and means for withdrawing overhead melamine off gas containing ammonia and carbon dioxide from said second reaction zone.

Said communication port has a liquid inlet section which, in operation, is located below the level of the liquid melamine melt contained in the first reaction zone.

The inlet section of the communication port is therefore immersed in the liquid melamine melt. A liquid head exists over said inlet section and, in operation, creates a hydraulic seal between the reaction zones.

The communication port may include a single passage or a plurality of passages.

Preferably all the liquid passages between the first reaction zone and the second reaction zone are located below the liquid level of melamine melt in the first reaction zone, i.e. there is no liquid passage between said two zones at or above said liquid level.

Said liquid level may be defined by the upper edge of an inner shell of the reactor which divides the first reaction zone from the second reaction zone. In some embodiments, said liquid level may be defined by a transition between different portions of said inner shell, for example a transition from a cylindrical portion to a conical portion. Accordingly the reactor may comprise one or more liquid passages located below said upper edge or transition of the inner shell.

In an embodiment, the separation means between the reaction zones include an internal shell and a cover. Said internal shell is coaxial to the outer pressure shell of the reactor and is arranged to separate the two reaction zones in the radial direction, so that the first reaction zone is inside the internal shell and the second reaction zone is delimited radially between said internal shell and the outer pressure shell of the reactor. For example the second reaction zone is an annular region between said inner shell and the outer pressure shell of the reactor.

Said cover may be located above the internal shell in a region of the reactor between the outer pressure shell and the internal shell. In addition, said cover may include passages for withdrawing the melamine offgas from the second reaction zone, and a lower portion extending in the first reaction zone and below an upper edge of the internal shell.

In this embodiment, a liquid passage is formed between the lower portion of the cover and the internal shell. At the lower edge of the cover, in particular, an inlet section for the liquid is formed, which is below the level of the melamine melt when, in operation, the inner reaction space is filled with liquid melamine.

More preferably, said lower portion of the cover may extend in the first reaction zone coaxially to an upper portion of the internal shell, so that an annular passage for the melamine melt is delimited between said lower portion of the cover and upper portion of the internal shell.

Preferably said internal shell is cylindrical and said lower portion of the cover is also cylindrical.

The passages for withdrawing the melamine offgas are preferably located in the proximity of the outer pressure shell. For example said passages may include suitable holes or slots.

In an embodiment, said separation means include an internal shell which is coaxial to the pressure shell of the reactor and includes a lower portion at a first radial distance from the outer pressure shell and an upper portion at a second radial distance from the outer pressure shell which is less than said first radial distance, the upper portion being therefore closer to the outer pressure shell than the lower portion. The internal shell also includes a transition portion connecting the lower portion with the upper portion.

A space for withdrawal of the melamine offgas is defined between the outer pressure shell and the upper portion of the internal pressure shell. As the upper portion of the internal shell is relatively close to the outer shell, said annular space may have a small gas passage cross section resulting in a high velocity of the gas with the advantages explained above. Particularly, the convective flow is increased and the undesired diffusive transfer of CO₂ is reduced thanks to the high velocity of the gas flow in this annular space.

The lower portion of the internal shell may include passages for the melamine melt from the first reaction zone to the second reaction zone, which are located below the level of the melamine melt in operation.

The lower portion and the upper portion of the internal shell may be cylindrical; the transition portion may be conical or substantially conical.

In another embodiment, an internal shell which divides the two reaction zones form one another may have an upper portion configured to cover the second reaction zone in a sealed manner. Accordingly the reactor includes suitable means for extraction of the offgas liberated in the second reaction zone, for example including nozzles on the outer pressure shell.

An advantage of this embodiment is a physical separation of the gas in the first reaction zone from the gas in the second reaction zone, to avoid the undesired diffusion of CO₂.

A process for the synthesis of melamine from urea according to the invention may be performed in a reactor comprising a first reaction zone and a second reaction zone, wherein the second reaction zone is an annular zone arranged coaxially around the first reaction zone.

The process includes:
feeding fresh urea into the first reaction zone, where a liquid crude melamine melt is formed with a high-pressure non-catalytic process;
transferring said melamine melt from the first reaction zone to the second reaction zone for further processing;
withdrawing a melamine-containing product from the second reaction zone, and withdrawing overhead melamine off gas containing ammonia and carbon dioxide from said second reaction zone;
wherein the crude melamine melt flows from the first reaction zone to the second reaction zone via one or more communication ports having a liquid inlet section in the first reaction zone which is located below the level of the liquid melamine melt contained in the first reaction zone.

The further processing of the melamine melt in the second reaction zone may include stripping the melamine melt with gaseous ammonia. The gaseous ammonia is then removed together with gaseous carbon dioxide as melamine offgas.

The invention is now further elucidated with the help of the drawings.

### Description of figures

Fig. 1 is a cross-sectional diagram of a combined primary and secondary melamine reactor of the prior art.
Fig. 2 is a detail of a combined reactor similar to Fig. 1 modified in according to a first embodiment of the invention.
Fig. 3 is an enlarged detail of Fig. 2.
Fig. 4 illustrates another embodiment of the invention.
Fig. 5 illustrates another embodiment of the invention.

### Detailed description

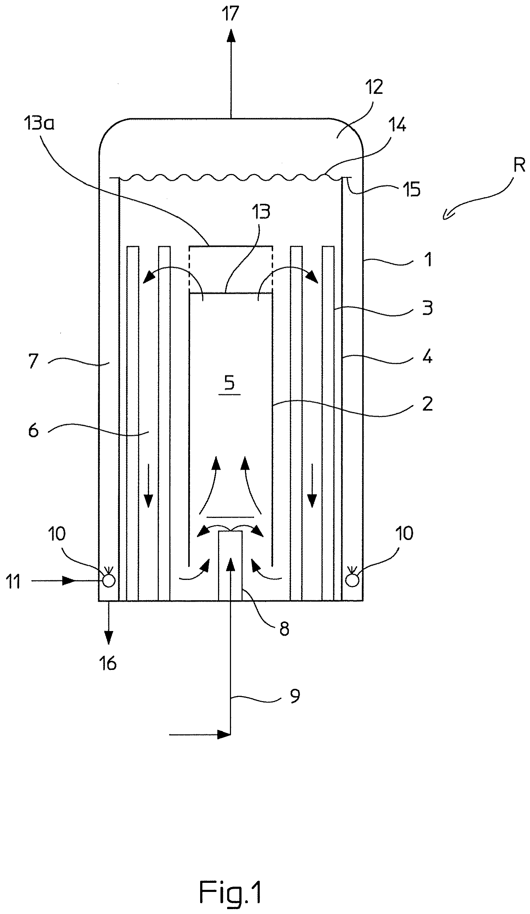

Fig. 1 illustrates a combined primary and secondary melamine reactor R with coaxial reaction chambers 6 and 7 separated by a shell 4.

Particularly, Fig. 1 illustrates the following details.
- 1: outer pressure vessel of the reactor (outer shell)
- 2: central duct
- 3: heating elements, e.g. heating pipes
- 4: inner shell
- 5: internal zone of the first reaction chamber, delimited by the central duct 2
- 6: peripheral zone of the first reaction chamber
- 7: second reaction chamber (annular reaction chamber)
- 8: urea melt inlet
- 9: urea melt feed
- 10: toroidal distributor
- 11: gaseous ammonia feed
- 12: gas separation chamber
- 13: open top section of the duct 2 with deflector 13a
- 14: liquid level of melamine during normal operation
- 15: edge of inner shell 4
- 16: melamine output
- 17: offgas discharge line

The inner shell 4 defines a first reaction chamber composed of an internal zone 5 delimited by the central duct 2 and a peripheral zone 6, outside the central duct 2, housing the heating pipes 3. A second reaction chamber 7, with a substantially annular shape, is delimited between said shell 4 and the outer shell 1.

In operation, the liquid melamine reaches the level denoted by the line 14 and flows over the top edge 15 of the inner shell 4 and into the annular chamber 7.

In the annular chamber 7 a stripping of the liquid melamine is performed by upraising gaseous ammonia introduced by the toroidal distributor 10 located at the bottom of the annular chamber 7. The purified melamine 16 thus obtained (after stripping) is discharged from the bottom of the chamber 7; the gases released during the stripping process and containing predominantly CO₂ and ammonia are collected in the chamber 12 and discharged via line 17.

This reactor of Fig. 1 (prior art) basically provides a primary conversion section in the chambers 5 and 6 and a secondary stripping section in the annular chamber 7. The crude melamine formed in the chambers 5, 6 is transferred to the chamber 7 for secondary processing (stripping) by overflowing from the inner shell 4.

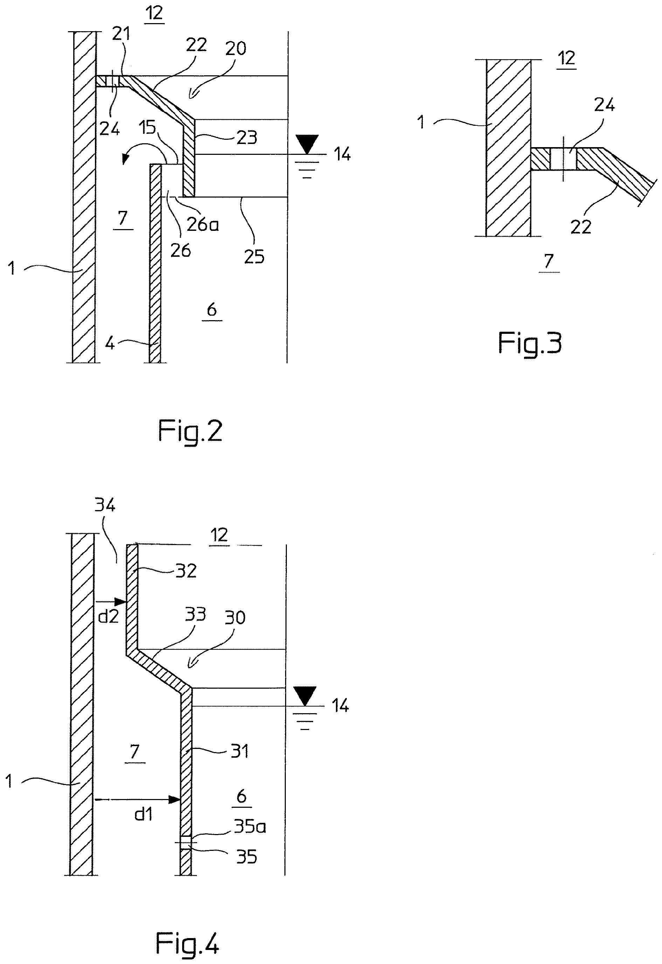

Fig. 2 illustrates a first embodiment of the invention. The same numerals of Fig. 1 are used for clarity.

According to Fig. 2, the reactor includes a cover 20 of the annular chamber 7 which is joined in a sealed manner to the inner surface of the outer shell 1.

Preferably the cover 20 has a first portion 21 joined to the outer shell 1, a second portion 22, a third portion 23.

The first portion 21 has one or more passages 24 for the offgas. Said passages 24 put the annular chamber 7 in a gas communication with the above gas collection chamber 12. The passages 24 may be holes or slots and are preferably located in the vicinity of the outer shell 1.

The third portion 23, which is a lower portion of the cover 20, extends in the first reaction zone 6 and below the upper edge 15 of the inner shell 4. Particularly, the lower edge 25 of the cover 20 (lower edge of its portion 23) is below the edge 15 of the inner shell 4.

The third portion 23 is distanced from the inner shell 4 so that a substantially annular passage 26 is delimited therebetween. Said annular passage 26 provides a communication port for the liquid melamine from the reaction chamber 6 to the reaction chamber 7.

In operation, the liquid melamine in the chamber 6 will reach a level 14 above the edge 15, due to the presence of the cover 20 and its portion 23. The height of the level 14 above the edge 15 provides the driving force for the melamine liquid to flow through the submerged passage 26 into the chamber 7. It can be noted that the passage 26 has an inlet section 26a which is submerged being below the melamine level 14.

The liquid melamine forms a liquid seal preventing the gas liberated in the second chamber 7 from flowing back into the first chamber 6. Said gas is collected in the upper chamber 12 via the passages 24. Additionally, the gas above the chamber 6 is prevented from entering the chamber 7 and diffuse in the liquid melamine contained therein.

The second chamber 7 is substantially separated from the gas collection chamber 12 by the cover 20 and the gas passage between said two chambers is possible only at the gas passages 24, which may be in a limited number and act substantially unidirectionally from chamber 7 to chamber 12. Therefore the collection of a gaseous phase in the top part of the second chamber 7 and above the liquid is avoided. Consequently, the possible diffusion of gaseous CO₂ into the liquid melamine contained in the second chamber 7 is avoided or greatly reduced.

Fig. 4 illustrates an embodiment wherein the separation between the first chamber 6 and the second chamber 7 is provided by an internal shell 30 which is coaxial to the outer shell 1 and replaces the previously described inner shell 4.

Said internal shell 30 includes a lower portion 31, an upper portion 32 and a transition portion 33. The lower portion 31 is at a first radial distance ***d1*** from the outer pressure shell 1 and the upper portion 32 is at a second radial distance ***d2*** from the same, wherein ***d2*** is smaller than ***d1**.* The upper portion 32 is therefore closer to the outer pressure shell 1.

An annular space 34 for withdrawal of the melamine offgas is defined between the outer pressure shell 1 and the upper portion 32 of the internal pressure shell 30. Preferably the distance ***d2*** is small, so that the annular space 34 has a relatively small cross section to increase the velocity of the gas flow in the space 34.

Similarly to the previously described embodiment, the chambers 7 and 12 are substantially separated by the shell 30 and a gas communication is only possible via the space 34. Due to the fast gas flow in this space 34, the convective flow of the gas leaving the chamber 7 prevails over the diffusive transfer and therefore the diffusion of CO₂ into the liquid contained in the chamber 7 is avoided or substantially reduced.

The transfer of the crude melamine from the chamber 6 to the chamber 7 is provided by a number of submerged holes 35 of the shell 30, more precisely of its lower portion 31. Each hole 35 has an inlet section 35a which is also below the liquid level 14.

In operation, the passages 35 are located below the liquid level 14 in the chamber 6. The maximum level to be reached by the liquid melamine may correspond, for example, to the transition between the portions 31 and 33 of the shell 30.

Preferably the lower portion and the upper portion of the internal shell 30 are cylindrical and the transition portion 33 is conical or substantially conical as illustrated.

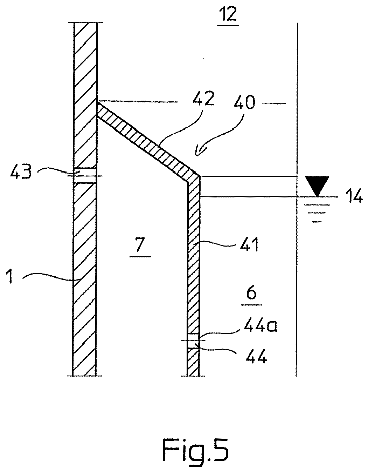

Fig. 5 illustrates another embodiment wherein the chambers 6 and 7 are separated by an internal shell 40 which includes: a lower portion 41 that radially separates said two chambers 6, 7 and an upper portion 42 configured to cover the chamber 7 in a sealed manner. In this case, the gas liberated in the chamber 7 is extracted via nozzles 43 of the outer pressure shell 1. The melamine melt is transferred from the chamber 6 into the chamber 7 by a number of submerged holes 44 with inlet sections 44a made in the lower portion 41 of said internal shell 40.

In this embodiment of Fig. 5, there is a physical separation between the reaction zones 6 and 7 to avoid the undesired diffusion of CO₂.

The nozzles 43 may be connected to an offgas extraction line. In an embodiment, said offgas extraction line may be provided with one or more flow control valves to regulate the pressure in the chamber 7.

In all the embodiments (e.g. embodiments of Figs. 2 to 5) the chamber 6 belongs to a first reaction zone which may also include an inner zone around the axis of the reactor delimited by a duct 5 as in Fig. 1.

The invention in its various embodiments achieves the goals enunciated above.

A liquid seal is established between the chambers 6 and 7, thanks to the fact that the liquid passages for the melamine melt to travel from the inner chamber 6 to the outer chamber 7 are below the level 14 in the chamber 6, i.e. a static head exists over the inlet of the liquid.

A difference of pressure is also created between the two chambers 6 and 7, the outer chamber 7 being at a greater pressure. Particularly, in the embodiments of Figs. 2 to 4 the greater pressure of the chamber 7 is due to a difference of pressure (Delta-p) which is necessary for the gaseous ammonia to flow through the relatively small cross section of the passages 24 or of the annular space 34. In other words it can be said that the pressure in the chamber 7 substantially equals the pressure in the chamber 12 plus the delta-p of the gaseous ammonia. In the embodiment of Fig. 5 the pressure in the chamber 7 may also be controlled by one or more flow regulation valves provided on an offgas extraction line connected to the nozzles 43.

The melamine offgas are extracted from the outer chamber 7, where stripping of the crude melamine melt occurs. A better separation between the offgas and the liquid contained in the outer chamber 7 is also obtained. The residence of a gaseous phase over the liquid in the outer chamber 7 and the undesired diffusion of CO₂ in the liquid are prevented. Accordingly the conversion of byproducts into melamine is facilitated, which ultimately results in a better purity (less content of byproducts) of the produced melamine 16.

## Claims

1. Reactor for the high-pressure non-catalytic synthesis of melamine from urea, comprising an outer pressure shell (1), a first reaction zone (6) and a second reaction zone (7) contained in said outer shell (1), wherein the second reaction zone is an annular space arranged coaxially around the first reaction zone, the reactor further including:
a urea input arranged to direct a urea feed into the first reaction zone, which is a zone for conversion of urea into a crude melamine melt;
separation means arranged to separate the first reaction zone from the second reaction zone;
at least one communication port (26, 35, 44) between the first reaction zone (6) and the second reaction zone (7) arranged to transfer the liquid crude melamine melt formed in the first reaction zone to the second reaction zone for further processing;
at least one melamine outlet for withdrawing a purified melamine-containing product from bottom of the second reaction zone, and at least one gas passage (24, 34, 43) for withdrawing overhead melamine off gas containing ammonia and carbon dioxide from said second reaction zone (7);
wherein said at least one communication port has a liquid inlet section (26a, 35a, 44a) which, in operation, is located below the level (14) of the liquid melamine melt contained in the first reaction zone.

2. Reactor according to claim 1, wherein said separation means include an internal shell (4) and a cover (20) wherein:
said internal shell is coaxial to the pressure shell of the reactor, arranged to separate the two reaction zones in a radial direction, so that the first reaction zone (6) is inside the internal shell and the second reaction zone (7) is delimited radially between said internal shell (4) and the outer pressure shell (1) of the reactor,
said cover (20) is located above the internal shell in the annular region between the outer pressure shell and said internal shell;
said cover having gas passages (24) for withdrawing the melamine offgas from the second reaction zone;
said cover having a lower portion (23) extending in the first reaction zone (6) and below an upper edge (15) of the internal shell (4).

3. Reactor according to claim 2, wherein said lower portion (23) of the cover (20) extends in the first reaction zone coaxially to at least an upper portion of the internal shell, so that an annular passage (26) for the melamine melt is delimited between said lower portion of the cover and upper portion of the internal shell.

4. Reactor according to claim 3, wherein said internal shell is cylindrical and said lower portion of the cover is also cylindrical.

5. Reactor according to any of claims 2 to 4, wherein said gas passages (24) of the cover for withdrawing the melamine offgas are located in the proximity of the outer pressure shell (1).

6. Reactor according to any of claims 2 to 5, wherein said gas passages of the cover include holes or slots.

7. Reactor according to claim 1 wherein said separation means include an internal shell (30) which is coaxial to the pressure shell (1) of the reactor and includes:
a lower portion (31) at a first radial distance from the outer pressure shell:
an upper portion (32) at a second radial distance from the outer pressure shell which is less than said first radial distance, the upper portion being therefore closer to the outer pressure shell than the lower portion;
a transition portion (33) connecting the lower portion with the upper portion.

8. Reactor according to claim 7, wherein an annular space (34) for withdrawal of the melamine offgas is defined between the outer pressure shell and the upper portion of the internal pressure shell.

9. Reactor according to claim 7 or 8, wherein said at least one communication port for the liquid crude melamine melt includes passages (35) provided in the lower portion (31) of said internal shell (30), which are located below the level (14) of the melamine melt in operation.

10. Reactor according to any of claims 7 to 9 wherein the lower portion and the upper portion of the internal shell are cylindrical, and the transition portion is conical or substantially conical.

11. Reactor according to claim 1 wherein said separation means include an internal shell (40) which is coaxial to the pressure shell of the reactor and includes:
a lower portion (41) that radially separates the first reaction zone from the second reaction zone;
an upper portion (42) configured to cover the second reaction zone in a sealed manner;
wherein the reactor further includes nozzles (43) on the outer pressure shell for withdrawing the melamine offgas from the second reaction zone.

12. Reactor according to claim 11, wherein said lower portion (41) of the internal shell (40) includes submerged passages (44) for the melamine melt from the first reaction zone to the second reaction zone.

13. Reactor according to any of the previous claims, including means for feeding gaseous ammonia to the second reaction zone.

14. A process for the synthesis of melamine from urea with a high-pressure non-catalytic process, wherein the process is performed in a reactor comprising a first reaction zone (6) and a second reaction zone (7), wherein the second reaction zone is an annular zone arranged coaxially around the first reaction zone, wherein the process includes:
feeding fresh urea into the first reaction zone, where a liquid crude melamine melt is formed;
transferring said crude melamine melt from the first reaction zone to the second reaction zone for further processing;
withdrawing a melamine-containing product from bottom of the second reaction zone, and withdrawing overhead melamine off gas containing ammonia and carbon dioxide from said second reaction zone;
wherein the melamine melt is transferred from the first reaction zone to the second reaction zone via one or more passages located below the level of the liquid melamine melt contained in the first reaction zone.

15. A process according to claim 14, wherein the further processing of the melamine melt which is performed in the second reaction zone includes stripping the melamine melt with gaseous ammonia.

16. A process according to claim 14 or 15 wherein the pressure in the second reaction zone (7) is greater than the pressure in the first reaction zone (6).
